# EUROPEAN PATENT APPLICATION

(11) **EP 3 984 504 A1**
(43) Date of publication of application: **20.04.2022**
(21) Application number: 20201616.8
(22) Date of filing: 13.10.2020
(51) Int. Cl.: A61F 9/007, A61B 17/122

(54) **SCLERAL DEVICE FOR TREATMENT OF OPEN ANGLE GLAUCOMA**

(71) Applicant: GlauVue, Inc., Meriden, CT 06450 (US)
(72) Inventor: LEVESQUE, Gaston, Meriden, CT Connecticut 06450 (US); CARO, Nicholas, Meriden, CT Connecticut 06450 (US); HARRISON, Marcie, Meriden, CT Connecticut 06450 (US)
(74) Representative: Pons

(57) **Abstract**

The present invention is directed to a scleral device for treatment of open angle glaucoma, configured to be attached to an eye and prevent loss of eye sight and blindness. Thanks to the scleral device the sclera is supported or reinforced, while the special relationship between the ciliary muscle and the lens is substantially unchanged. The scleral device (1) comprises a curved central portion (8), two arms (11) which extends from the ends (9) relatively movable with respect to each other from an open position to a closed position, wherein the arms (11) have a first sector (12) and a second sector (13) extending from the first sector (12), which comprises a hook (14) in a free end configured to grasp the sclera tissue (5) in a closed position.

## Description

### OBJECT OF THE INVENTION

The present invention is directed to a scleral device for treatment of open angle glaucoma, configured to be attached to an eye and to prevent loss of eyesight and blindness. Thanks to the scleral device the sclera is supported and reinforced, while the special relationship between the ciliary muscle and the lens remain substantially unchanged. For this purpose, incisions in the conjunctiva have to be made to gain access to the sclera overlying the ciliary muscle.

### BACKGROUND OF THE INVENTION

Glaucoma is a vision disorder associated with excess intraocular pressure in the eye. Intraocular pressure is a function of production of liquid aqueous humor by the ciliary processes of the eye, and its drainage through the trabecular meshwork. Aqueous humor flows from the ciliary processes into the posterior chamber, and it flows through the pupil of the iris into the anterior chamber, bounded posteriorly by the iris and anteriorly by the cornea. From here, the trabecular meshwork drains aqueous humor via the scleral venous sinus into scleral plexuses and general blood circulation.

In open/wide-angle glaucoma, flow is reduced, due to the degeneration and obstruction of the trabecular meshwork, whose original function is to absorb the aqueous humor. Loss of aqueous humor absorption leads to increased resistance and thus a build-up of pressure in the eye.

### DESCRIPTION OF THE INVENTION

These objects, as well as others which will become apparent upon reference to the following detailed description and accompanying drawings, are accomplished by a scleral device for treatment of open angle glaucoma, configured to be attached to an eye having an iris, a ciliary muscle, a scleral tissue and a conjunctiva covering the scleral tissue and comprising at least one tunnelled incision adjacent to the iris that overlies the ciliary muscle, wherein the scleral device is symmetrical with respect to a longitudinal axis and is configured to be partially housed in the tunnelled incision and to be linked to the scleral tissue and comprises a curved central portion comprising two ends and an inner face having a concave curvature adapted to the curvature of the eye and configured to contact the scleral tissue and two arms which extends from the ends relatively movable with respect to each other from an open position to a closed position. Each arm comprises a L-shaped longitudinal section formed by a first sector which extends outwardly from the end in opposite direction to the inner face, and a second sector extending from the first sector, which is longer than the first sector, which comprises a hook in a free end configured to grasp the sclera tissue in a closed position.

The scleral device supports and reinforces the sclera, while substantially maintaining the special relationship between the ciliary muscle and the lens. Before placing the scleral device, incision in the conjunctiva has to be done, to gain access to the sclera overlying the ciliary muscle. The tenon's capsule are moved laterally to expose the sclera, and the sclera is extended outwardly. A scleral device, or various scleral devices, are provided having two arms for engaging the outwardly extended sclera there between. The arms of the scleral device are extended in the sclera so as to excerpt pressure on the sclera, and then the tenon's capsules are slid over the scleral device and the conjunctiva is closed.

This mechanism is analogous to how the drug pilocarpine works. Pilocarpine reduces intraocular pressure (IOP) by inducing contraction of the ciliary muscle. The mechanism of IOP reduction is mediated by increased tension exerted by the longitudinal ciliary muscles at its circumferential insertion on the scleral spur. The scleral device assists the ciliary muscles to facilitate the flow of aqueous fluid through the trabecular meshwork. The scleral device treats glaucoma by supporting and reinforcing the sclera with a removable device.

The scleral device is attached to the sclera by first making an incision, tunnelled incisions, in the conjunctiva to gain access to the sclera's overlying the ciliary muscle. The Tenon's capsule is moved laterally to expose the sclera and the sclera is incised appropriately. The arms of the device engage a portion of the sclera resulting in compression over the ciliary body. The Tenon's capsule covers the device and the conjunctiva is closed.

The scleral device restores the level of force which the ciliary muscle exerts on the trabecular meshwork, thus opening the drainage pores and relieving the intraocular pressure (IOP). The scleral device decreases the production and increases the outflow of aqueous fluids through the trabecular meshwork. The decrease in production results from compression of the ciliary muscle. The increase in outflow occurs due to the anatomical changes induced by the scleral device to treat Open Angle Glaucoma.

The scleral device strengthens the sclera and assist in maintaining the spatial relationship between the sclera and the ciliary system. The material of the scleral device can be metallic machined or Metal Injected (MIM) or non-metallic machined or moulded.

The central portion and the first sector may be joined by a first curved junction and the first sector and the second sector may be joined by a second curved junction.

The distance between the first junction and the curved configuration of an external face, opposite to the inner face, in this area, once inserted in the sclera give an outwardly pressure to the scleral device pushing the arms into tunnelled incisions of the sclera resulting in downward constant pressure on the eye from the inner face.

As mentioned, first of all, an incision in sclera are made and then the surgical pocket is made. The arms are inserted in the sclera incision and twisted in surgical pocket. Then, in order to insert the second arm in sclera, the device has to be bended 90° and pushed in sclera incision and then the arm will follow in surgical pocket. The surgical pockets are cavities in the sclera.

The thickness of the curved junctions is wider than the rest of the scleral device, wherein the rest of the scleral device may have a constant thickness.

The hook preferably comprises a narrow sector limited by two cut-outs defined on each side of the longitudinal axis and a wide sector, extending from the narrow sector which comprises two flaps on each side of the longitudinal axis wherein said flaps are limited by a curved outer edge.

The flaps secure the device and prevent slippage of the scleral device, thereto, and the Tenon's capsules are slid over the scleral device and the conjunctiva is closed. Preferably, up to four such scleral device is applied to the sclera substantially equally spaced about the lens between the medial, inferior, lateral and superior rectus muscle. When applied to the sclera, the scleral device serves to prevent the sclera from buckling under tension applied by the ciliary muscle.

The narrow sector give flexibility to the wide sector and the outer edge ease the insertion in the surgical pocket.

The first sector may form a first angle with the central portion bigger than 90° and the second sector forms a second angle with the first sector bigger than 90°.

The scleral device has a length of no more than 6.0mm and no less than 2.5mm so as to fit between adjacent rectus muscles. The arms contain but not limited to the flaps but may also be provided with means, such as barbs, teeth or spurs, for grasping but not penetrating the sclera. The arms of the scleral device remain in the closed position in the absence of an external force applied therebetween. The incisions for are positioned centred between the ciliary muscles at a depth no more than 260 microns and pocket length between 0.3mm and 0.5mm. The distance from the limbus to the incision may vary depending on the diameter of the eye and the limbus. The parallel incision accommodates the variable size of the scleral device have to be parallel incision between 2.5mm and 6.0mm with a tunnelling incision between 0.3mm and 0.5mm

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1.- is a front view of an eye with four scleral devices.
Figure 2.- is a side view of the scleral device.
Figure 3.- is a simplified diagram showing two scleral devices attached to an eye.
Figure 4A.- is a top view of the scleral device in an open situation.
Figure 4B.- is a section view of the scleral device.
Figure 5.- is a perspective view of a positioning ring.
Figure 6.- is a top view of the scleral device in a closed situation.

### PREFERRED EMBODIMENT OF THE INVENTION

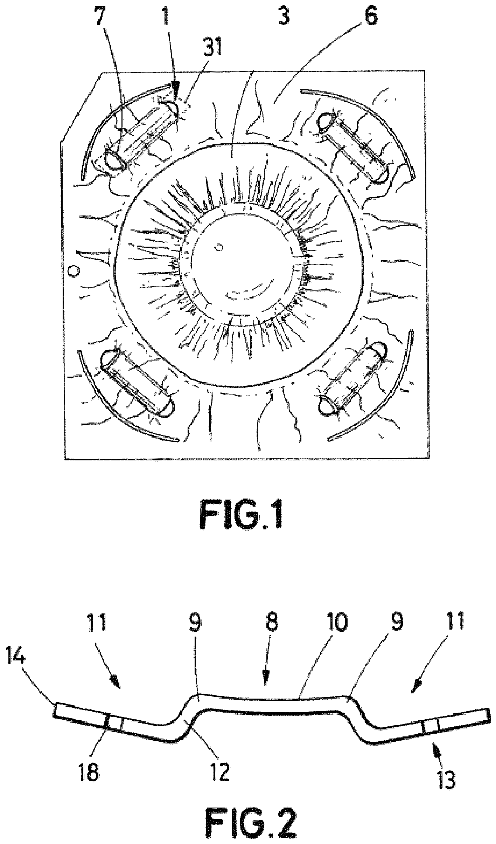

Figure 1, is a front view of an eye (2) with four scleral devices (1), according to the present invention, wherein the scleral devices (1) for treatment of open angle glaucoma, are attached to the eye (2) having a iris (3), a ciliary muscle (4) and a scleral tissue (5) and a conjunctiva (6) covering the scleral tissue (5) wherein the conjunctiva (6) comprises four tunnelled incisions (7) adjacent to the iris (3) that overlie the ciliary muscle (4). The scleral device (1) comprises two arms (11) and is configured to be partially housed in the tunnelled incision (7) and to be linked to the scleral tissue (5). In the preferred embodiment there are 4 scleral devices (1) surrounding the iris (3) arranged equidistant from each other.

Preferably, first of all two surgical pockets (31) are made, followed by tunnelled incisions (7) that connect the surgical pockets (31), for the placement of the surgical devices in the eye (2). First of all, one of the arms (11) is inserted in the tunnelled incision (7) and twisted to be introduced in one surgical pocket (31). Then, the other arm (11) has to be bended and pushed into the tunnelled incision (7) and placed in the other surgical pocket (31).

Figure 2 is a side view of the scleral device (1), wherein the scleral device (1) is symmetrical with respect to a longitudinal and transversal axis and comprises a curved central portion (8) comprising two ends (9) and an inner face (10) having a concave curvature adapted to the curvature of the eye (2) and the inner face (10) is configured to contact the scleral tissue (5) once the tunnelled incision (7) has been made in the conjunctiva (6) to gain access to the scleral tissue (5).

The scleral device (1) comprises two arms (11) which extends from the ends (9) relatively movable with respect to each other from an open position to a closed position. Each arm (11) comprises a L-shaped longitudinal section formed by a first sector (12) which extends outwardly from the end (9) in opposite direction to the inner face (10), and a second sector (13) extending from the first sector (12), which is longer than the first sector (12) and which comprises a hook (14) in a free end configured to grasp the sclera tissue (5) in a closed position.

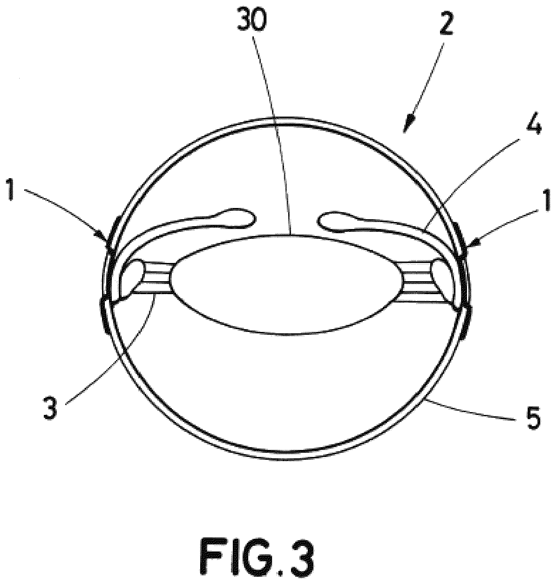

Figure 3 is a simplified diagram showing two scleral devices (1) attached to an eye (2), according to the present invention, wherein the devices (1) grasp the sclera tissue (5) overlying the ciliary muscle (4) which are placed adjacent to the iris (3) and connected to a lens capsule (30). The incisions (7), not shown, are positioned at a depth less than 260 microns and with a pocket length between 0.3mm and 0.5mm. The device is (1) is partially inserted in sclera tissue (5) and covered by conjunctiva (6), not shown.

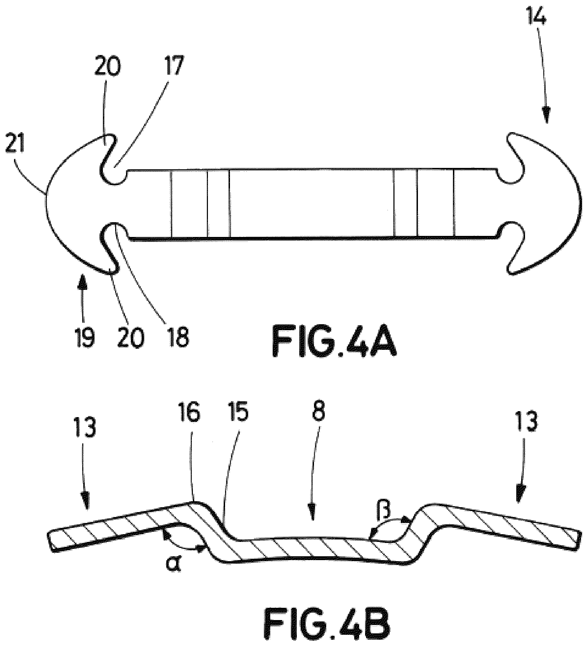

Figure 4A is a top view of the scleral device (1), in an open situation, according to the present invention, wherein each hook (14) comprises two cut-outs (17) on each side according to the longitudinal axis of the scleral device (1) that defined a narrow sector (18). The two cut-outs (17) are curved. The device (1) also comprises a wide sector (19), attached to the narrow sector (18), comprising two flaps (20) on each side according to the longitudinal axis of the scleral device (1) and it is defined by a curved outer edge (21). The distance between the ends (9) varies from 2.5 mm to 6.0 mm. In the figure, the scleral device (1) is in an open position and the hook are fold out, after being inserted.

Figure 4B is a section view of the scleral device, according to the present invention, wherein the central portion (8) and the first sector (12) are joined by a first curved junction (15) and the first sector (12) and the second sector (13) are joined by a second curved junction (16).The thickness of the curved junctions (15,16) is wider than the rest of the scleral device (1) and the rest of the scleral device (1) has a constant thickness. The first sector (12) forms a first angle (β) with the central portion (8) which is obtuse and the second sector (13) forms a second angle (α) with the first sector (12) which in a preferred embodiment is obtuse.

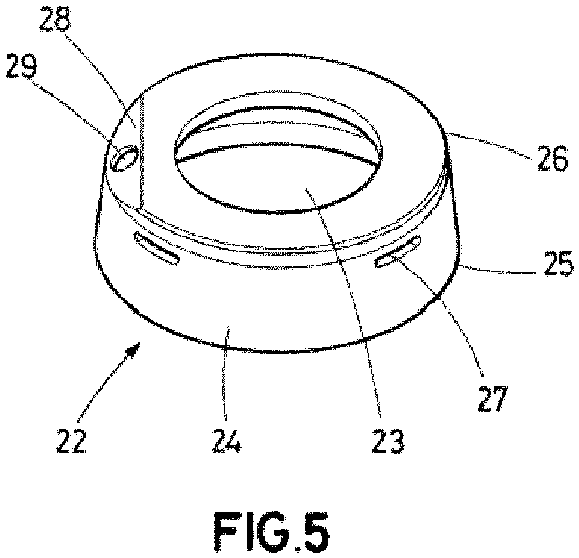

Figure 5 is a perspective view of a positioning ring for marking the position of the incisions (7), wherein the positioning ring comprises a cone shaped main body (22) with a through cavity (23) intended to partially house the iris (3) of the eye (2), where the main body (22) is defined by a perimeter surface (24) comprising a lower edge (25) intended to contact conjunctiva (6), an upper edge (26) and 4 through slots (27) near the upper edge (26) arranged equidistant from each other with possibility of housing a marking element to mark the position of the tunnelled incisions (7). The positioning ring comprises a chamfered area (28) on the upper edge with a hole (29) designed to accommodate a vacuum port from a suction device. A marker fits in the slots (27) and marks the sclera.

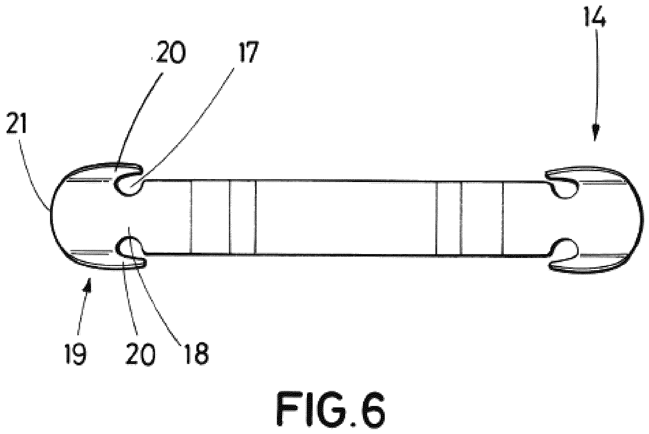

Figure 6 is a top view of the scleral device (1) in a close situation or locked situation, where the hooks are closed and facilitate the insertion of the device (1).

## Claims

1. Scleral device (1) for treatment of open angle glaucoma, configured to be attached to an eye (2) having an iris (3), a ciliary muscle (4), a scleral tissue (5) and a conjunctiva (6) covering the scleral tissue (5) and comprising at least one tunnelled incision (7) adjacent to the iris (3) that overlies the ciliary muscle (4), wherein the scleral device (1) is symmetrical with respect to a longitudinal axis and is configured to be partially housed in the tunnelled incision (7) and to be linked to the scleral tissue (5) and comprises:
- a curved central portion (8) comprising two ends (9) and an inner face (10) having a concave curvature adapted to the curvature of the eye and configured to contact the scleral tissue (5);
- two arms (11) which extends from the ends (9) relatively movable with respect to each other from an open position to a closed position,
**characterized in that** each arm (11) comprises a L-shaped longitudinal section formed by:
a first sector (12) which extends outwardly from the end (9) in opposite direction to the inner face (10), and
a second sector (13) extending from the first sector (12), which is longer than the first sector (12), which comprises a hook (14) in a free end configured to grasp the sclera tissue (5) in a closed position.

2. The scleral device of claim 1, wherein the central portion (8) and the first sector (12) are joined by a first curved junction (15) and the first sector (12) and the second sector (13) are joined by a second curved junction (16).

3. The scleral device of claim 1, wherein the thickness of the curved junctions (15,16) is wider than the rest of the scleral device (1).

4. The scleral device of claim 1, wherein the hook (14) comprises a narrow sector (18) limited by two cut-outs (17) defined on each side of the longitudinal axis and a wide sector (19), extending from the narrow sector (18) which comprises two flaps (20) on each side of the longitudinal axis wherein said flaps are limited by a curved outer edge (21).

5. The scleral device of claim 1, wherein the first sector (12) forms a first angle (β) with the central portion (8) which is obtuse.
